# EUROPEAN PATENT SPECIFICATION

(11) **EP 0 478 587 B1**
(45) Date of publication and mention of the grant of the patent: **26.10.1994**
(21) Application number: 90908569.8
(22) Date of filing: 04.06.1990
(51) Int. Cl.: A61F 2/38

(54) **IMPROVEMENTS IN AND RELATING TO KNEE PROSTHESES**
KNIEPROTHESE
AMELIORATIONS PORTANT SUR LES PROTHESES DU GENOU

(30) Priority: 02.06.1989 GB 8912682
(43) Date of publication of application: 08.04.1992
(73) Proprietor: CHAS. F THACKRAY LIMITED, Leeds LS6 2DP (GB)
(72) Inventor: ELLOY, Martin, Arthur, Church Fenton North Yorkshire (GB); JOHNSON, Robert, Merseyside (GB)
(74) Representative: Browne, Robin Forsythe, Dr.
(86) International application number: GB9000861
(87) International publication number: WO9014806

(56) References cited:
- DE-A- 2 631 351
- FR-A- 2 290 883
- GB-A- 2 070 939
- US-A- 4 822 365

## Description

The present invention relates to an improved artificial knee prosthesis for use in the aforesaid method.

A knee prosthesis according to the features of the preamble of Claim 1 is known from FR-A-2290883.

Artificial knee prostheses generally comprise a femoral component for attachment to the femur, a tibial component for attachment to the tibia, and in some cases there is also a meniscal component for location between the femoral and tibial components.

Conventional knee prostheses of this type are manufactured as complete knees in a range of different sizes, each knee comprising femoral, meniscal and tibial components of matching size, to suit different patients. The surgeon must plan the knee replacement operation in advance, deciding on the size of prosthesis which he will require during surgery, from radiographs taken prior to surgery. This is quite a difficult task and does not allow the surgeon to change his mind during the course of surgery should he find that a different size of prosthesis is required.

According to the present invention there is provided a set of components from which a knee prosthesis may be constructed, the set comprising a plurality of femoral components each having a bone engaging surface for attachment to a femur and a bearing surface for bearing engagement with a corresponding bearing surface of another component of a knee prosthesis, at least one tibial component having a bone engaging surface for attachment to a tibia and a bearing surface for bearing engagement with a corresponding bearing surface of another component of a knee prosthesis, and at least one meniscal component having a first bearing surface for bearing engagement with said bearing surface of said tibial component and a second bearing surface for bearing engagement with said bearing surface of said femoral component, characterised in that all of the femoral components of the set have identical bone engaging surfaces but different sizes and/or shapes of bearing surfaces.

Thus, because the profile of all of the bone engaging surfaces of the different femoral components is the same, the surgeon does not need to decide beforehand which size of prosthesis is required. He merely resects the femur to a standard size and shape and then selects, during surgery, the most appropriate size of femoral component.

Preferably the set also includes a plurality of tibial components each having a bearing surface of different size and/or shape. The bearing surface of the tibial component is generally referred to as "the tibial plateau", and in a preferred embodiment of the invention, as well as providing tibial components with plateaux of different size and/or curvature, there are also provided tibial components with different thicknesses of tibial plateau, as measured along the longitudinal axis of the extended leg.

To match the different shapes and sizes of femoral and tibial components there is also preferably provided a plurality of meniscal components each having first and second bearing surfaces of different size and/or shape. The meniscal components are also conveniently provided in different thicknesses.

Embodiments of the present invention will now be described by way of example only with reference to the accompanying drawings in which:

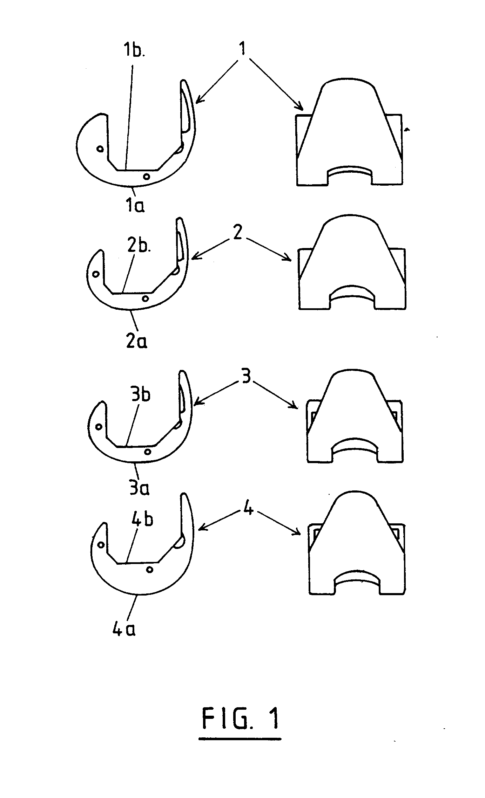

Figure 1 illustrates the different shapes and sizes of femoral component;
Figure 2 illustrates the different shapes and sizes of tibial component;
Figure 3 illustrates the different shapes and sizes of meniscal component;
Figure 4 illustrates meniscal subluxation caused by incorrect tension in the anterior-posterior (A-P) plane;
Figure 5 illustrates mensical subluxation due to valgus/varus instability;
Figure 6 illustrates the equalisation of the flexed and extended gaps between the cut surfaces of the tibia and the femur;
Figure 7 illustrates distraction of the flexed knee prosthesis;
Figure 8 illustrates the extension of the prosthesis;
Figure 9 illustrates the situation where the knee will not fully extend;
Figure 10 illustrates the use of the large femoral component to reduce the flexion gap;
Figures 11 and 12 illustrate the use of two different sizes of meniscal component in flexion (Figure 11) and extension (Figure 12) to calculate the amount of bone that needs to be removed from the distal femur;
Figures 13 and 14 illustrate the use of a saw guide to remove different thicknesses of bone from the distal femur;
Figures 15 and 16 illustrate the restoration of valgus/varus stability;
Figures 17, 18 and 19 illustrate the technique of soft tissue release;
Figure 20 illustrates the resection of the natural patella;
Figure 20a illustrates a patella clamp/drill for drilling the resected patella;
Figure 21 illustrates the use of a femoral pusher to hold the femoral component in place whilst hardening of the bone cement takes place;
Figure 22 illustrates a modified tibial component for restaining subluxation at the meniscal-femoral interface;
Figures 23 and 24 illustrate a modified femoral component for use with the meniscal component of Figure 23;
Figure 25 illustrates a peg for achieving constraint at the menisco-tibial interface.

Referring to the drawings, the set of components includes a series of different femoral components 1,2.3 and 4. Component 1 is designated "large", 2 is designated "standard", 3 is designated "small" and 4 is designated "extra small". A greater range of sizes may be provided as convenient.

Each of these components has a different bearing surface 1a, 2a, 3a and 4a, but they all have identical bone engaging surfaces 1b, 2b, 3b and 4b.

Also included in the set are a number of different tibial components 5, 6, 7 each having a different bearing surface 5a, 6a, 7a but having identical tibial shanks 5b, 6b, 7b for implantation in the tibia. Tibial component 5 is designated "large", 6 is designated "standard" and 7 is designated "small". Each size is also supplied with a thicker tibial plateau, as designated by reference numerals 8, 9 and 10.

There are also three sizes of meniscal components 11 (large), 12 (standard) and 13 (small) to match the different sizes of femoral and tibial component. Each size may be availble in several different thicknesses as measured along the long axis of the extended leg.

The technique of implantation of the artificial knee will now be described in detail. First of all the surgeon resects the femur, preferably using the intramedullary alignment system and instruments described in the applicants' own pending European patent application No 86300525.2. to achieve a distal femoral profile which corresponds to the bone engaging surfaces of all of the femoral components in the set.

The surgeon then prepares the tibia using the method and instruments disclosed in the aforementioned European patent application.

After preparing the femur and tibia, trial femoral and tibial components (17,18) are pushed into position but not cemented in place.

The next stage of the procedure is the restoration of soft tissue stability.

With an unconstrained prosthesis such as that described herein, it is essential to achieve correct soft tissue balance in both the A-P and the valgus/varus planes. The soft tissue sleeve of the extensor mechanism, posterior capsule and collateral ligaments must be equally tensioned otherwise subluxation of the meniscus can occur.

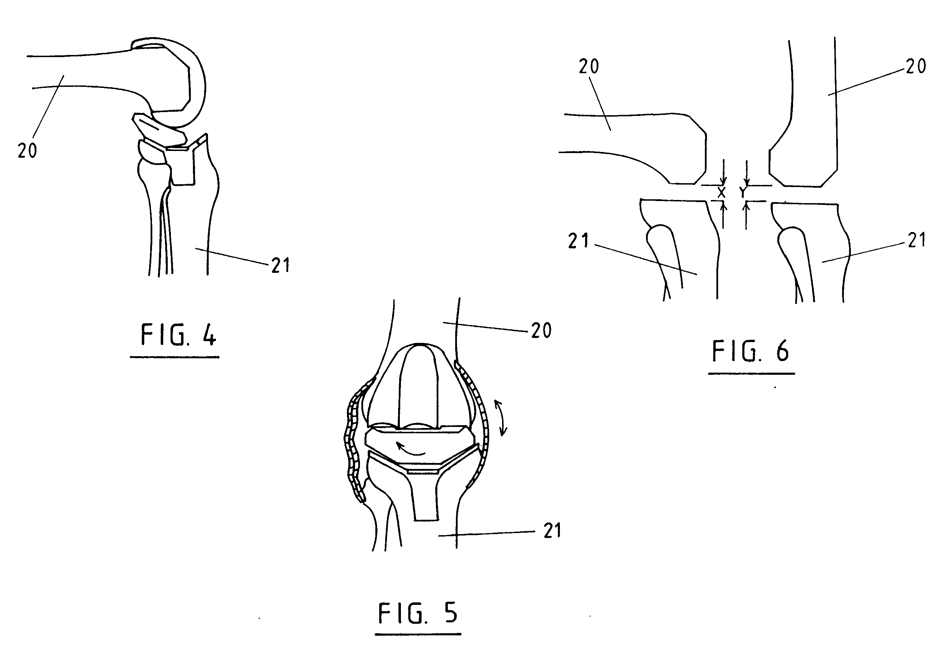

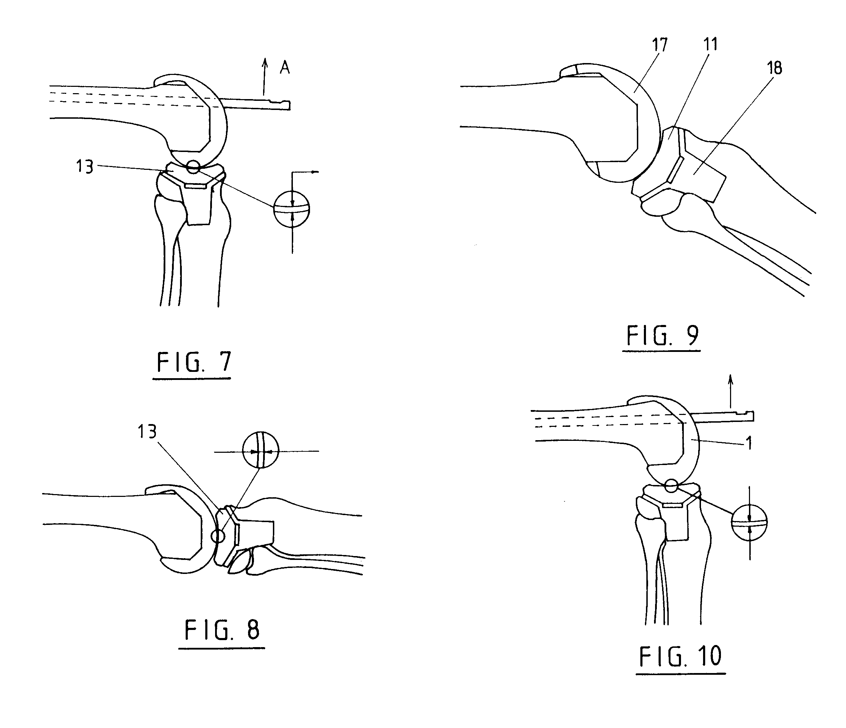

If correct tension in the A-P plane is not achieved then the femur rides forward on the tibia causing meniscal subluxation, as shown in Figure 4. Valgus/varus inequality will cause subluxation away from the tight collateral ligaments, as shown in Figure 5.

It is essential not to do any soft tissue releases until all the bone cuts are made and the trial components are in place. Even in very gross deformities the collateral ligaments may be of equal tension and it is impossible to predict pre-operatively which patients will require releases.

The restoration of A-P stability is achieved by equalising the flexed and extended gaps (X,Y respectively) between the cut surfaces of the tibia 21 and the femur 20, ensuring that the collateral ligaments are equally taut in both flexed and extended position, as shown in Figure 6 from which the ligaments have been omitted for the sake of clarity.

With the knee in 90^{o} flexion and the trial components in place, the knee is distracted in the direction shown by arrow A in Figure 7, using the protruding end of the alignment rod and the foot.

Starting with the thinnest meniscal component 13 so as not to over distract the collateral ligaments, a suitable thickness of meniscus is inserted such that no laxity exists. The correct tension is when the femur 20 will not sublux anteriorly or posteriorly on the meniscus when pulled forwards or backwards at 90^{o} of flexion. The knee is now extended and will fall into full extension in approximately 90% of cases, as shown in Figure 8.

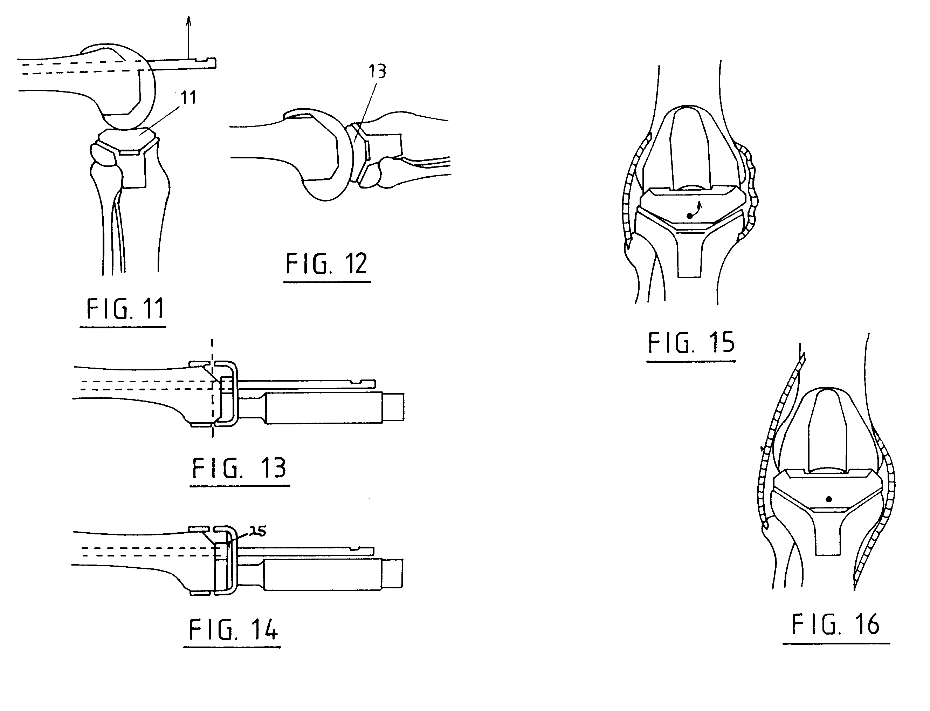

Differences in the flexion/extension gap X,Y occur in approximately 10% of patients, the commonest situation being that the flexion gap X is larger than the extension gap Y. In this case, with the thick meniscus 11 in flexion, the knee will not fully extend, as shown in Figure 9. Soft tissue balance can be restored by:-
(A) Release or division of the posterior capsule, but this is potentially dangerous and is not recommended.
(B) Trying the large femoral component 1 which has 6mm more metal posteriorly but the same amount of metal distally compared to the standard size 2. This effectively reduces the flexion gap by 6mm, as shown in Figure 10.
(C) Removal of bone from the distal femur. The amount of bone that needs to be resected from the distal femur can be checked using the correct meniscal size in extension and subtracting this from the meniscal size in flexion. This will be in multiples of 3mm. In the example shown in Figure 11 a 9mm gap is present in flexion and only 3mm in extension. Thus, 6mm of bone needs to be removed from the distal femur. To remove more bone from the distal femur the angle adaptor is placed over the rod onto the cut surface of the femur. With the second saw guide in place 9mm of bone will be re-sected (Figure 13). Using the 3 and 6mm shims between the angle adaptor and saw guide. different thicknesses of bone can be removed (Figure 14). There is however a limit to the amount of bone that can be re-sected because of the attachments of the collateral ligaments to the femur.

The next stage of the operation is the restoration of valgus/varus stability, and this is illustrated in Figures 15 and 16. With the knee in 90^{o} of flexion, a thickness of meniscus is inserted such that there is no laxity. The knee is then extended. If there is a significant difference between the tension of the collateral ligaments, the meniscus will sublux away from the tight side, as shown in Figure 15. When the meniscus stops dislocating soft tissue tension is equalised and collateral ligament releases are stopped at this stage, as shown in Figure 16.

Next, the technique of soft tissue release will be described in detail, dealing first of all with medial soft tissue release.

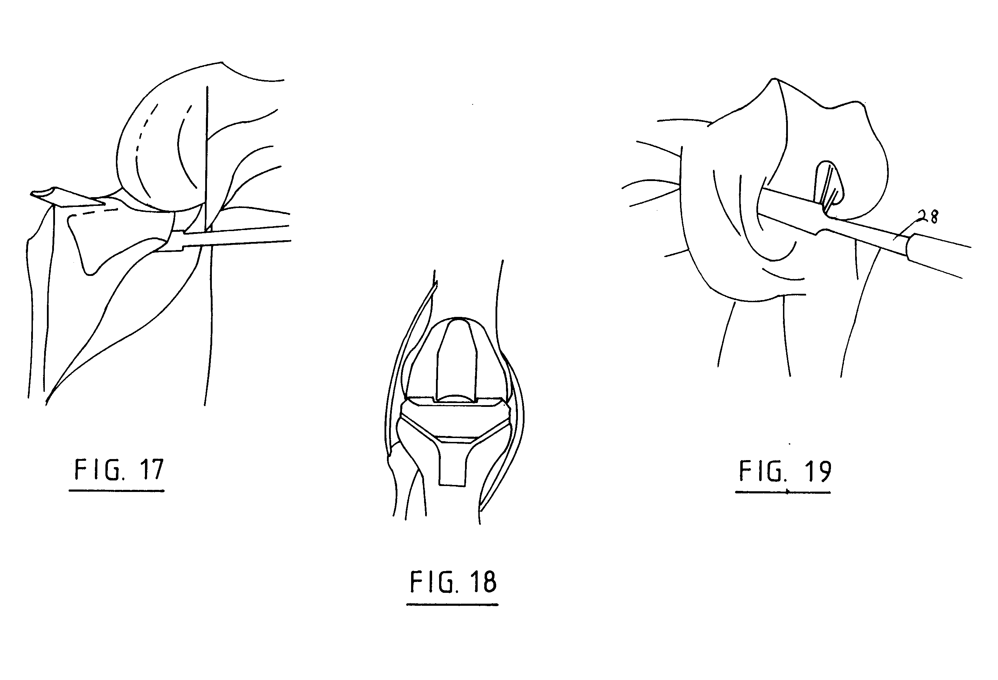

All osteophytes are removed from the femur and tibia prior to ligament release, including the flare of the medial tibial plateau. Next, the tightest part of the ligament is defined. This is often postero-medial, and a small subperiosteal release may be sufficient to restore balance. Then, using a bone lever, the medial collateral ligament can sometimes be stretched sufficiently to stop meniscal subluxation, as shown in Figure 17. A subperiosteal release of the soft tissue sleeve from the upper medial tibia is carried out until meniscal subluxation stops, as shown in Figure 18.

Lateral soft tissue release is then carried out, bearing in mind that the lateral structures may cause lateral popliteal nerve palsy and if a large deformity is to be corrected then the nerve should be released. First of all, all osteophytes are removed from the femur and tibia. The popliteus tendon is then removed, and the lateral ligament is released from the femur. Lateral ligament release is best achieved using a osetotome 28 to remove the boney attachment of the ligament, as shown in Figure 19, which can there be stretched until meniscal subluxation stops. Finally, if deformity is still present the ilio-tibial band is divided. Often with severe deformities requiring lateral release a patella retinacular release is required at the same time because of the lateral position of the patella.

If a significant release of either the medial or lateral structures is required, it is often necessary to impose some constraint on the prosthesis, and this can be achieved using a smaller constraining peg in the tibial component, or the femoral constraining box, or both. These special devices are described in more detail below.

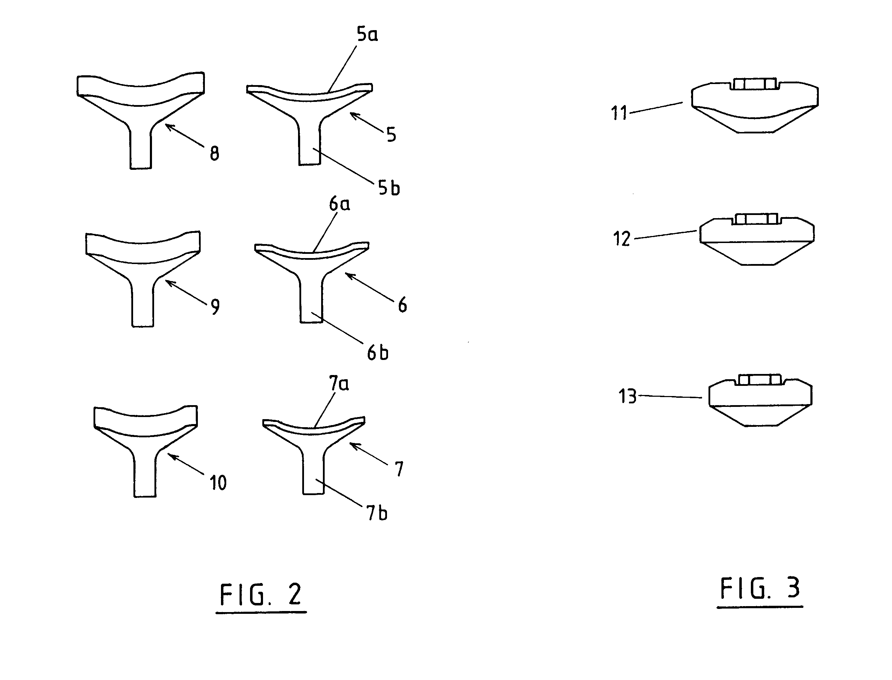

The next stage is the permanent fixation of the tibial component, the surgeon having first selected the desired tibial component from the range shown in Figure 2. Generally, the largest tibial component is used, and the thicker tibial components are used if the boney gaps are large.

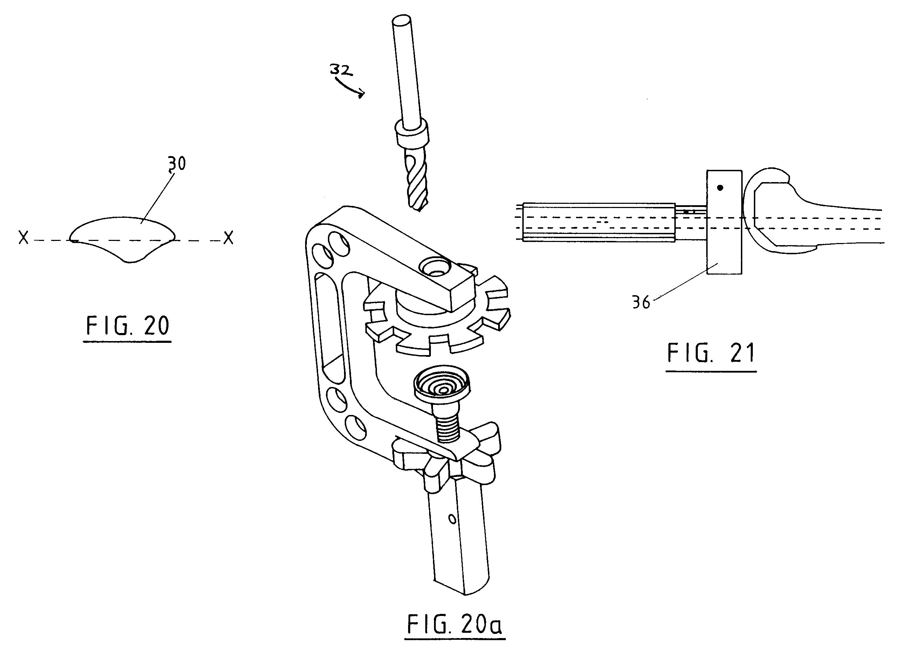

Having secured the tibial component in the tibia using bone cement, the natural patella 30 is resected freehand along line X-X as shown in Figure 20. Then, using the patella clamp/drill 32, shown in Figure 20a, a hole may be drilled in the resected patella for a central peg of a patella button which is to be implanted. The patella button also comes in a range of sizes, and the correct size of patella is chosen at this stage.

Next, the selected femoral component is secured to the femur by pressing cement into the grooves on the fixation surfaces of the femoral prosthesis, which is then passed over the femoral alignment rod and bone and then tapped into position using the femoral pusher 36, as shown in Figure 21. Excess cement is removed, particularly from the intercondular gap and the back of the joint. The pusher is used to maintain pressure until the cement has hardened. Some of the femoral mix of cement is used to fix the patella button, which is held in place using the patella clamp whilst femoral cementation is completed. Since the femoral saw cuts are the same for all femoral components, this enables the surgeon to change the size of the femoral component during the operation without having to change the femoral cuts. Mix and match is possible with, for example, small femoral and large tibial components. The meniscal size is related to the tibial size, and the meniscal component must never be larger than the tibial size. The components may be secured without use of cement, for example by utilisation of fixing pegs disclosed in the prior art. Fixing pegs may be provided with serrations to prevent detatchment of the implanted prosthesis.

Prosthetic fixation in true alignment is now complete, and it only remains to restore full stability by fitting the appropriate meniscus. Trial reduction should be repeated at this stage to check that there has been no change in the size of the meniscus required. The knee should then be thoroughly cleaned and all cement particles removed before the definitive meniscus is inserted. Finally, a check should be made that the meniscus does not impinge on any bone or soft tissue in the intercondular area, and, if necessary, bone or soft tissue is removed.

The tracking of the patella component is checked, and it should run over the femoral condyles with no tension laterally. If there is any tension at all, a lateral patella retinacular release is carried out.

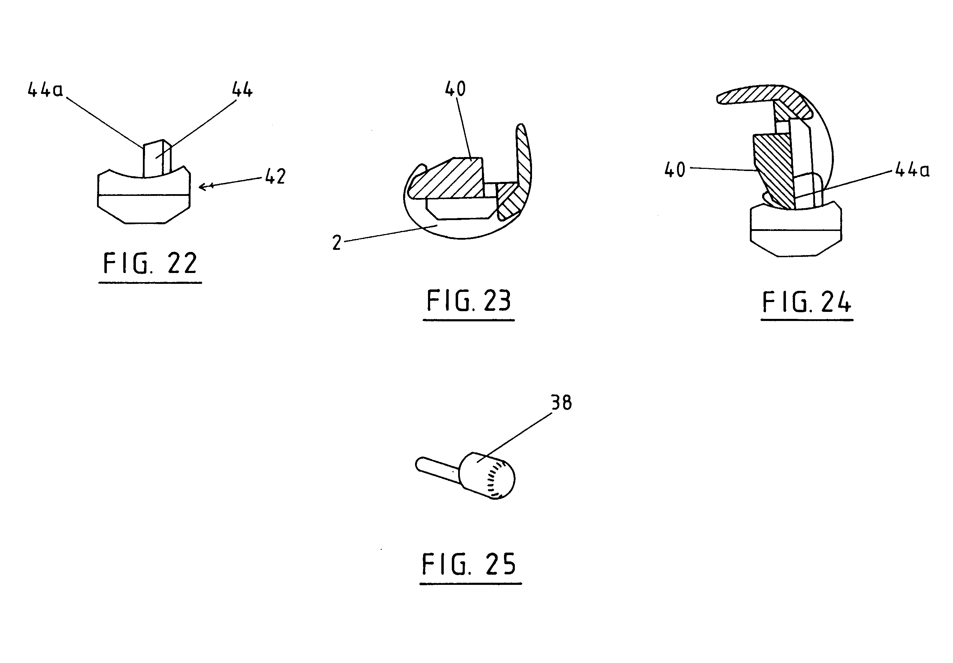

In cases where ligament stability cannot be achieved, constraint at the menisco-tibial interface can be achieved by the introduction of the large or small constraining peg 38 shown in Figure 25. The small peg allows 15^{o} of subluxation in all directions, whereas the large peg allows no subluxation but full rotation.

Further constraint at the menisco-femoral interface can be achieved by the use of the femoral constraining box 40, which can be locked on to the standard femoral component by use of a small instrument. The use of the box is illustrated in Figures 22, 23 and 24. The meniscal component 42 shown in Figure 22 includes an upwardly standing post 44, the posterior surface 44a of which serves to engage the femoral constraining box when the knee is at or near full flexion, as shown in Figure 24.

Forward movement of the femoral component is prevented by abutment. The box shaped construction of the femoral component allows use of a large post but prevents tissue ingrowth into the cavity.

The box may incorporate a stem inclined at the valgus angle of 7^{o} and adapted to be inserted into the femoral cavity. The stem may be provided as a separate module which is preferably reversible to facilitate use in left or right knees. A series of stems of different lengths may be provided.

Fixation pegs and serrated pads may be provided as disclosed in our EP 186471.

## Claims

1. A set of components from which a knee prosthesis may be constructed, the set comprising a plurality of femoral components (1,2,3,4) each having a bone engaging surface (1b,2b,3b,4b) for attachment to a femur and a bearing surface for bearing engagement with a corresponding bearing surface of another component of a knee prosthesis, at least one tibial component having a bone engaging surface for attachment to a tibia and a bearing surface (5a, 6a, 7a) for bearing engagement with a corresponding bearing surface of another component of a knee prosthesis, and at least one meniscal component (11;12;13) having a first bearing surface for bearing engagement with said bearing surface of said tibial component and a second bearing surface for bearing engagement with said bearing surface of said femoral component, characterised in that all of the femoral components of the set have identical bone engaging surfaces but different sizes and/or shapes of bearing surfaces.

2. A set of components according to claim 1 comprising a plurality of tibial components (5,6,7) each having a bearing surface (5a,6a,7a) of different size and/or shape.

3. A set of components according to claim 2 comprising tibial components with different thickness of tibial plateau (8,9.10) as measured along the longitudinal axis of an extended leg.

4. A set of components according to any one of the preceding claims comprising a plurality of meniscal components (11,12,13) each having first and second bearing surfaces of different size and/or shape.

5. A set of components according to claim 3 wherein the meniscal components are provided in different thicknesses.

## Patentansprüche

1. Satz von Komponenten, aus denen eine Knieprothese herstellbar ist, mit einer Mehrzahl femoraler Komponenten (1, 2, 3, 4), die jeweils eine Knochenanlagefläche (1b, 2b, 3b, 4b) zur Befestigung an einem Femur und eine Tragfläche zur tragenden Anlage an einer entsprechenden Tragfläche einer anderen Komponente der Knieprothese aufweisen, mit mindestens einer tibialen Komponente, welche eine Knochenanlagefläche zur Befestigung an einem Schienbein und eine Tragfläche (5a, 6a, 7a) zur tragenden Anlage an einer entsprechenden Tragfläche einer weiteren Komponente der Knieprothese aufweist, und mit mindestens einer miniskalen Komponente (11; 12; 13), die eine erste Tragfläche zur tragenden Anlage an der Tragfläche der tibialen Komponente und eine zweite Tragfläche zur tragenden Anlage an der Tragfläche der femoralen Komponente aufweist, dadurch gekennzeichnet, daß alle femoralen Komponenten des Satzes identische Knochenanlageflächen jedoch unterschiedliche Größen und/ oder Formen von Tragflächen aufweisen.

2. Satz von Komponenten nach Anspruch 1, dadurch gekennzeichnet, daß er eine Mehrzahl tibialer Komponenten (5, 6, 7) umfaßt, die jeweils eine Tragfläche (5a, 6a, 7a) unterschiedlicher Größe und/oder Form aufweisen.

3. Satz von Komponenten nach Anspruch 2 dadurch gekennzeichznet, daß er tibiale Komponenten mit unterschiedlicher Dicke des tibialen Plateaus (8, 9, 10), gemessen entlang der Längsachse eines ausgestreckten Beines, umfaßt.

4. Satz von Komponenten nach einem der vorhergehenden Ansprüche, dadurch gekennzeichnet, daß er eine Mehrzahl meniskaler Komponenten (11, 12, 13) umfaßt, die jeweils eine erste und eine zweite Tragfläche unterschiedlicher Größe und/oder Form aufweisen.

5. Satz von Komponenten nach Anspruch 3, dadurch gekenn kennzeichnet, daß die meniskalen Komponenten in unterschiedlichen Dicken vorgesehen sind.

## Revendications

1. Ensemble de composants à partir duquel une prothèse du genou peut être construite, l'ensemble comprenant plusieurs composants fémoraux (1, 2, 3, 4), présentant chacun une surface s'engageant avec l'os (1b, 2b, 3b, 4b) destinée à être fixée sur un fémur et une surface de support destinée à s'engager en la supportant avec une surface de support correspondante d'un autre composant d'une prothèse du genou, au moins un composant tibial présentant une surface s'engageant avec l'os destinée à être fixée à un tibia et une surface de support (5a, 6a, 7a) destinée à s'engager en la supportant avec une surface de support correspondante d'un autre composant d'une prothèse du genou, et au moins un composant méniscal (11, 12, 13) présentant une première surface de support destinée à s'engager en la supportant avec ladite surface de support dudit composant tibial et une seconde surface de support destinée à s'engager en la supportant avec ladite surface de support dudit composant fémoral, caractérisé en ce que tous les composants fémoraux de l'ensemble présentent des surfaces s'engageant avec l'os identiques, mais des surfaces de support de taille et / ou de forme différente.

2. Ensemble de composants selon la revendication 1, comprenant plusieurs composants tibiaux (5, 6, 7) présentant chacun une surface de support (5a, 6a, 7a) de taille et / ou de forme différente.

3. Ensemble de composants selon la revendication 2, comprenant des composants tibiaux dont le plateau tibial présente des épaisseurs différentes (8, 9, 10) lorsqu'il est mesuré le long de l'axe longitudinal d'une jambe étendue.

4. Ensemble de composants selon l'une des revendications précédentes, comprenant plusieurs composants méniscaux (11, 12, 13) présentant chacun des première et seconde surfaces de support de taille et / ou de forme différente.

5. Ensemble de composants selon la revendication 3, dans lequel les composants méniscaux sont fournis en différentes épaisseurs.
